# EUROPEAN PATENT SPECIFICATION

(11) **EP 2 858 617 B1**
(45) Date of publication and mention of the grant of the patent: **16.08.2023**
(21) Application number: 12740653.6
(22) Date of filing: 12.06.2012
(51) Int. Cl.: A61F 13/66, A61F 13/471, A61F 13/505, A61F 13/49, A41B 9/02

(54) **MEN'S BRIEFS WITH SEPARATE SPACE FOR PENIS COMPRISING REMOVABLE INCONTINENCE ABSORBENT RECEPTACLE**
MÄNNERSLIP MIT ABGETRENNTEM RAUM FÜR DEN PENIS UND MIT ABNEHMBAREM HARNINKONTINENZABSORPTIONSBEHÄLTER
SLIPS POUR HOMMES AVEC ESPACE SÉPARÉ POUR LE PÉNIS COMPRENANT UN RÉCEPTACLE ABSORBANT AMOVIBLE POUR L'INCONTINENCE

(43) Date of publication of application: 15.04.2015
(73) Proprietor: NEORES d.o.o., 40315 Mursko Sredisce (HR)
(72) Inventor: Delija, Frane, 21000 Split (HR)
(74) Representative: Sucic, Tatjana
(86) International application number: PCT/HR2012/000014
(87) International publication number: WO 2013/186577

(56) References cited:
- WO-A1-86/05387
- WO-A1-88/06008
- WO-A1-89/00037
- WO-A1-2012/013991
- GB-A- 2 173 990
- US-A- 4 695 279
- US-A- 5 074 853
- US-A- 5 275 592
- US-A- 5 707 364
- US-A- 5 722 127
- US-A1- 2005 015 067
- US-A1- 2005 033 258
- US-A1- 2006 276 764
- US-A1- 2011 077 610
- US-B1- 6 569 135

## Description

### TECHNICAL FIELD

The present invention relates to men's underwear, in particular men's underpants having separate space to physically separate the penis from the testicles at the inner side of their front part, comprising a removable incontinence absorbent receptacle and to the incontinence absorbent receptacle as such.

### TECHNICAL PROBLEM AND BACKGROUND ART

The penis and the testicles are constantly in contact, which is not good from the hygienic and health points of view. Due to their position, these organs are constantly exposed to sweating. Particularly negative effect is from the drops of urine remaining after urination or caused by problems with mild incontinence.

The present invention aims to provide versatile men's briefs especially suitable for wear by active persons who suffer from mild urinary incontinence or for patients recovering after surgery. Moreover, it is an advantage of the arrangements disclosed herein that the briefs are intended for those not incontinent and for those suffering incontinence.

The technical problem that is solved by this invention relates to designing of men's briefs, where the penis will be physically separated from the testicles, comprising a removable incontinence absorbent receptacle adapted to fit within said men's briefs in an easy and comfortable way with tightening means for preventing the penis to go beyond the incontinence absorbent receptacle.

Since the penis is naturally slightly directed to the left or right side, the present invention provides such natural position avoiding any pressure caused by briefs. On the other hand, an overlapping inner insert prevents falling out of the penis from the briefs separate space, especially during long-lasting sitting position. A further advantage of the present invention is that the briefs' orifice is oriented left or right, depending on the customer preferences.

A further advantage of the present invention is adjustably tightening of the penis thus preventing the penis to go beyond the incontinence absorbent receptacle during sleep or any other sport activity.

Document GB 2174288 A discloses a pair of men's briefs (200) includes a pocket located in the crotch region (212) and defined by an outer wall of a liquid-impermeable material which is longer than it is wide and an inner wall of like material which has a central hole therein, and the outer and inner walls are positioned so as to retain, in use, an elongate absorbent pad in the pocket. According to present invention briefs orifice has circular like form which encircles penis in a way that prevents its falling out from separated space, and furthermore present invention provides elastic means for adjustably tightening of penis. Further advantage over document GB 2174288 A relates to the material of the briefs and form of the incontinence absorbent receptacle. According to the GB 2174288 A absorbent pad is in elongate form and crotch region of the briefs is made by liquid-impermeable material.

Document WO2012/013991 A1 is closest prior art and discloses Men's briefs with a separated space for penis, made as short or long leg briefs, with a flexible belt, a panel, a front outer insert and a front inner insert, wherein the separated space for the penis is formed by the front outer insert and the front inner insert; the inserts (B) and (B') being connectable to the panel and an optional part in a manner to form men's briefs, wherein the front inner insert has shape and dimensions same as the front outer insert. The men's brief, according to the present invention, is further provided with a removable incontinence receptacle, where the removable incontinence receptacle has shape same as said inserts and is placed within front outer insert and front inner insert.

Document US2006/276764 A1 discloses urinary incontinence device which is comfortable and provides desired pressure control for restricting urine flow. The moisture-absorbing layer defined as an elongated hollow sleeve for receiving the user's penis therein. the incontinence loop cinch may be carried between the inner and outer surfaces. The incontinence loop cinch may also be carried adjacent the outer surface. A clamp may be carried by the incontinence loop cinch for holding the incontinence loop cinch in the cinched position.

Document WO86/05387 A1 discloses an incontinence appliance for a man consists of a pouch which is made of a water-impermeable material and is lined with a layer of absorbent material. The layer of absorbent material is in a form of a segment of such a size and shape that the entire inner surface of the pouch is covered by the layer of absorbent material. The pouch is adapted to fit loosely around the penis.

Document US6569135 B1 is related to a urine absorbent pouch for male incontinence which completely surrounds the patient's penis, thereby minimizing the patient's exposure to wetness.

Document WO88/06008 A1 is related to a disposable absorbent article such as a diaper or a sanitary napkin, comprising an absorbent body which is encased in an envelope which comprises two surface layers which are joined mutually around the absorbent body and of which layers one is liquid impermeable and the other is liquid permeable.

According to the present invention parts of the briefs are not made of a liquid-impermeable material. Further, the absorbent article is in the form of a receptacle; it can be disposable or washable and comfortably fits within briefs. Further, since the receptacle according to the present invention is in the form of a three-dimensional container, it ensures retention of the urine within the receptacle and prevents contact of urine with briefs. That is very important in case where an absorbing layer of the receptacle is not able to absorb large quantity of urinated liquid.

The men's briefs with separate space for the penis adapted for incorporating a removable incontinence absorbent receptacle according to the present invention makes for a very useful part of underwear, aimed to the man's health, that can be manufactured very economically and in numerous embodiments. It includes essential improvements relative to previously known briefs and incontinence absorbents of this type.

### DETAILED DESCRIPTION OF THE INVENTION

The essence of the invention is a men's briefs with a separate space for a penis placed at the inner side of their front part and a removable incontinence absorbent receptacle which is adjustably placed within the separate space for penis.

The men's briefs with the separated space for receiving the penis comprising a removable incontinence absorbent receptacle, made as short or long leg briefs, with a flexible belt, a panel, a front outer insert and a front inner insert, wherein the separated space for the penis is formed by the front outer insert and the front inner insert; said inserts being connected to the panel and an optional part in a manner to form men's briefs, wherein the front inner insert has shape and dimensions same as the front outer insert. The front inner insert is formed from two overlapping sewed parts each having a curved hem, where the curved hems form a circular like adjustable orifice for inserting the penis into the separated space. The adjustable orifice is placed in the brief's midline, and said adjustable orifice encircles the penis in a way that prevents its falling out from separated space. The adjustable orifice is placed in the briefs' center line and is oriented on the left or right side by rotating the front inner insert by 180° around the brief's midline. Additionally, the curved hems have an elastic means for adjustably encircling the penis, where, according to one embodiment of the present invention, said elastic means can be tightened by the means on the outer side of the briefs. The shape of the adjustable orifice for entering the penis and the height at which it is made are adjusted to allow the penis to smoothly enter its separate space when the briefs are being put on. This way, the testicles remain in their normal position, always physically separated from the penis. The men's briefs are further provided with the removable incontinence receptacle, in a form of a three-dimensional container ensuring retention of the urine within the removable incontinence receptacle, having an orifice for receiving the penis, where the incontinence receptacle is placed within the front outer insert and the front inner insert, whereby the removable incontinence receptacle has a shape same as said inserts and has dimensions to fit within said inserts. According to the one embodiment of the present invention, the removable incontinence receptacle is disposable. According to another embodiment of the present invention the removable incontinence receptacle is washable and can be reused.

### Detailed description of drawings

invention will be below described in detail with reference to drawings where:
figure 1. shows a back and side panel A of the briefs,
figure 2. shows a front outer insert B,
figure 3. shows an outer front view of the briefs,
figure 4. shows view of a part C of an inner panel,
figure 5. shows view of a part D of an inner panel,
figures 5a. and 5b show view of the front inner insert B' with an orifice 19 left or right oriented,
figure 6a. schematically shows a removable incontinence receptacle (G) with an orifice 22,
figure 6b. schematically shows dismantled removable incontinence receptacle (G) with an orifice 22,
figure 7a shows a front side of the removable incontinence receptacle with an U-shaped orifice, and
figure 7b schematically shows an outer liquid barrier cover and placing inner absorption receptacle within it.

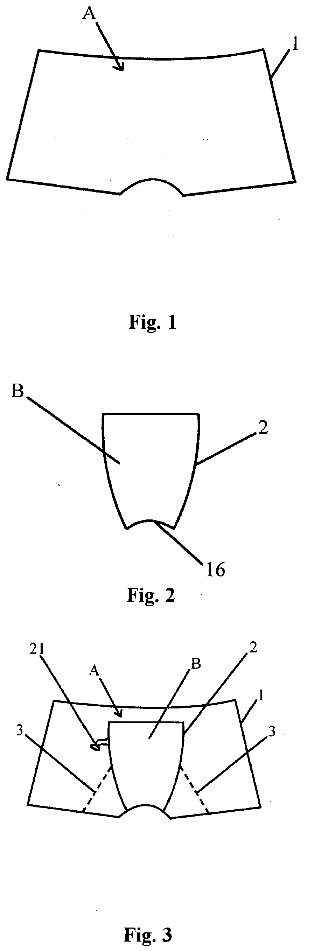

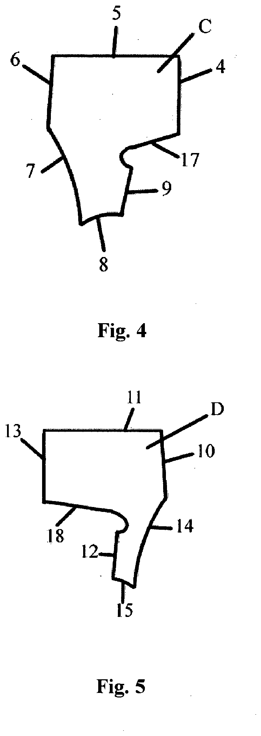

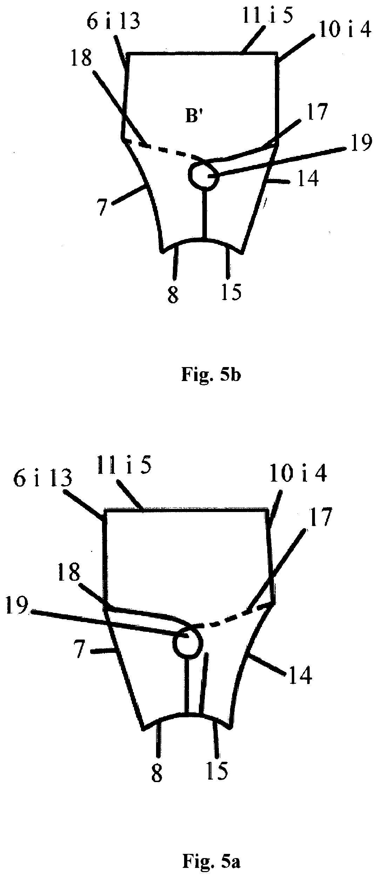

With reference to figures 1 to 5b, a men's briefs with a separated space for penis, made as short or long leg briefs, with a flexible belt, a panel (A), a front outer insert (B) and a front inner insert (B'), wherein the separated space for the penis is formed by the front outer insert (B) and the front inner insert (B'); the inserts (B) and (B') being connectable to the panel (A) and an optional part 3 in a manner to form men's briefs, wherein the front inner insert (B') has a shape and dimensions same as the front outer insert (B). The front inner insert (B') is formed from two overlapping sewed parts (C) and (D) having a curved hem 17 and a curved hem 18, where curved hems 17 and 18 form a circular like adjustable orifice 19 for inserting the penis into the separated space. As illustrated on figures 5a and 5b,according to the embodiment of the invention, it is provided that part (C) overlaps part (D) or part (D) overlaps part (C), without any further modification of said parts (C) and (D) and other parts of briefs. Figures 5a and 5b illustrate the adjustable orifice 19 placed in the briefs center line, and where the adjustable orifice 19 encircles the penis in a way that prevents its falling out from the separated space and its facilitates stable positioning. By rotating the front inner insert (B') by 180° around the briefs midline, the adjustable orifice 19 can be left or right side oriented thus enabling more comfortable placing of the penis on the left or right side according to the user's preferences. Additionally, curved hems 17 and 18 have an elastic means for adjustably encircling the penis, where, according to one embodiment of the present invention, said elastic means can be tightened by a means 21.

As shown on figures 4 and 5, the front inner insert (B') is formed from two parts (C) and (D) with mutually sewed hems 6 and 13, 8 and 16, 15 and 16, 9 and 12, where curved hems 17 and 18 form the circular like adjustable orifice 19 for inserting the penis into the separated space, where the adjustable orifice 19 is placed in the briefs midline, wherein the adjustable orifice 19 encircles the penis in a way that prevents its falling out from the separated space. With reference to the figures 5a and 5b, the front inner insert (B') being sewed with to the inner side of the outer panel (A) and the front outer insert (B) at least through hem 7 and hem 14, and through sewing hem 8 and hem 15 to hem 16. Furthermore, inserts (B) and (B') are connected to the outer panel (A) through hems 6 and 13, 10 and 4 and 8 and 15. The outer panel (A) constitutes the back side and partially the front side of the briefs. The outer panel (A) can be tailored in one piece where that one piece includes part of the front panel 3. For a person skilled in the art is obvious that certain modifications of the design of the outer panel (A) and inserts (B, B') are possible without impact on the scope of the invention. For instance, the outer panel (A) can be tailored from more than one piece of fabric (e.g. part 3 can be separate part of the briefs).

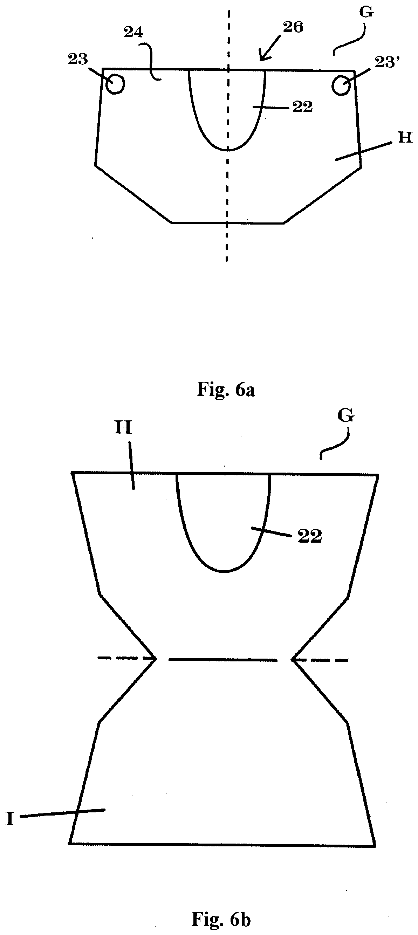

With reference to figures 6a to 7b, the men's briefs are further provided with a removable incontinence receptacle (G) having an orifice 22. The removable incontinence receptacle (G) is placed within the front outer insert (B) and the front inner insert (B') through an embrasure formed between hems 11 and 5. The removable incontinence receptacle (G) has shape same as inserts (B, B') and has dimensions to fit within inserts (B, B') in a so manner that the orifice 22 is faced to the adjustable orifice 19 of the man's briefs in order that the penis may pass through orifices 22 and 19 and may be accommodated within the removable incontinence receptacle (G). The expression "dimensions to fit within", and other variants of said expression, in the present application mean that the dimensions of an element to which said expression relates are such that it can be inserted or positioned within a defined space and can not be displaced from its position after insertion. The best mode of carrying out the invention is that the removable incontinence receptacle (G) has the same shape as inserts (B, B'), and dimensions to fit within inserts (B, B'), and the orifice 22 is faced to the adjustable orifice 19. The hems 17 and 18 have an elastic means for adjustably encircling the penis and additionally the elastic means can be tightened by the means 21. These features provide preventing the penis to go beyond the incontinence absorbent receptacle during sleep or intense activities.

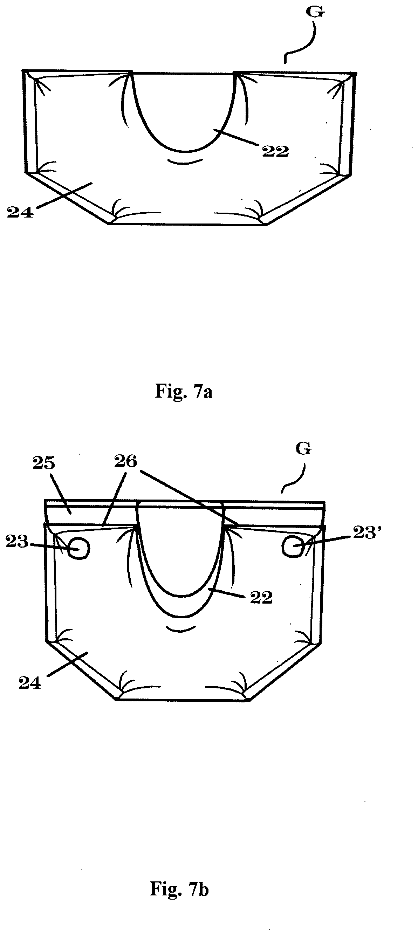

As shown in the figures 7a, 7b the removable incontinence receptacle (G) consists of an outer liquid barrier cover 24, and an inner absorption receptacle 25 having shape same as the liquid barrier cover 24 and dimensions to fit within the liquid barrier cover (24). The inner absorption receptacle 25 is placed within the liquid barrier cover 24, whereat the cover 24 and the inner absorption receptacle 25 are both provided with concurring orifices 22 at the same place. The orifice 22 can be circular or U-shaped or of any other shape suitable for receiving the penis. The orifice 22 is placed in the center line of the removable incontinence receptacle (G). The removable incontinence receptacle (G) is defined by a receptacle front panel (H) and a receptacle back panel (I) shown in figure 6b. Panels (H) and (I) are on the edges mutually firmly connected. In another embodiment, on an upper end the liquid barrier cover 24 is provided with an opening 26 for inserting the inner absorption receptacle 25. The inner absorption receptacle 25 is defined by two planes mutually firmly connected on their edges forming a pocket like receptacle. According to one embodiment of the invention, the removable incontinence receptacle (G) is disposable and consists of the outer liquid barrier cover 24 and the inner absorption receptacle 25, which liquid barrier cover 24 and the inner absorption receptacle 25 are on their edges mutually firmly connected. In this embodiment, the liquid barrier cover 24 is not provided with the opening 26. In another embodiment of the invention, the removable incontinence receptacle (G) is not disposable where the liquid barrier cover 24 and the inner absorption receptacle 25 are not mutually firmly connected thus enabling changing and/or washing of the inner absorption receptacle 25.

In order to additionally fix the removable incontinence receptacle (G) within the inserts (B, B'), they are provided with detachable means 23 and 23'. By means 23, 23', the removable incontinence receptacle (G) is detachably connected to the front inner insert (B') and/or front outer insert (B).

The men's briefs with a separate space for the penis comprising a removable incontinence absorbent receptacle, according to this invention, eliminates deficiencies of the present briefs cuts, related to their hygienic and health properties and physical discomforts. The separate space for the penis is situated at the inner side of the front part of the briefs, between the front outer insert and the front inner insert. The removable incontinence absorbent receptacle is adjustably placed within the separate space for the penis. Regardless of the body movements and position, the penis will always remain in the separate space and within the incontinence absorbent receptacle, which fully satisfies the essence and the function of the invention.

### REFERENCE SIGNS:

The reference signs used in the description and figures have the following meaning:
A - back and side panel of briefs
B - front outer insert
B'- front inner insert
C - part of the inner panel
D - part of the inner panel
G - removable incontinence receptacle
I - receptacle back panel
H - receptacle front panel
1 - panel A hem
2 - insert B hem
3 - optional part of the front panel
4 - hem sewed to hem 10
5 - hem
6 - hem sewed to hem 13
7 - sewed to inner side of the outer panel
8 - hem sewed to hem 16
9 - hem sewed to hem 12
10 - hem sewed to hem 4
11 - hem
12 - hem sewed to hem 9
13 - hem sewed to hem 6
14 - hem sewed to hem inner side of the outer panel
15 - hem sewed to hem 16
16 - hem sewed with hems 8 and 15
17. - curved hem
18 - curved hem
19 - adjustable orifice
20 - seam
21 - means suitable for adjustment of adjustable orifice 19
22 - orifice
23, 23' - detachable means
24 - outer impermeable cover - outer liquid barrier cover
25 - inner absorption receptacle
26 - opening

## Claims

1. A men's briefs with a separated space for receiving the penis comprising a removable incontinence absorbent receptacle (G), made as short or long leg briefs, with a flexible belt, a panel (A), a front outer insert (B) and a front inner insert (B'), wherein the separated space for receiving the penis is formed by the front outer insert (B) and the front inner insert (B'); the front outer insert (B) and the front inner insert (B') being connected to the panel (A) and an optional part (3) in a manner to form the men's briefs, wherein the front inner insert (B') has shape and dimensions same as the front outer insert (B), where the front inner insert (B') is formed from two overlapping sewed parts (C) and (D) having a curved hem (17) and a curved hem (18), respectively, the curved hems (17) and (18) have an elastic means for adjustably encircling the penis, where said curved hems (17) and (18) form a circular like adjustable orifice (19) for inserting the penis into the separated space, the adjustable orifice (19) encircles the penis in a way that prevents its falling out from the separated space, **characterized in that** said men's briefs are further provided with the removable incontinence receptacle (G) being in the form of a three-dimensional container ensuring retention of the urine within the removable incontinence receptacle (G), the removable incontinence receptacle (G) is defined by a receptacle front panel (H) and a receptacle back panel (I) that are on the edges mutually firmly connected and with an orifice (22) placed on the receptacle front panel (H), the removable incontinence receptacle (G) is placed within the front outer insert (B) and the front inner insert (B') through an embrasure formed by hems (11, 5), whereby the removable incontinence receptacle (G) has a shape same as said front outer and front inner inserts (B, B') and, as defined in the description, dimensions to fit within said front outer and front inner inserts (B, B') in a manner that the orifice (22) of the receptacle front panel (H) is faced to the adjustable orifice (19) in order that the removable incontinence receptacle (G) receives the penis, wherein the removable incontinence receptacle (G) consists of an outer liquid barrier cover (24) and an inner absorption receptacle (25), the inner absorption receptacle (25) is defined by two planes mutually firmly connected on their edges forming a pocket like receptacle, the inner absorption receptacle (25) having shape same as the outer liquid barrier cover (24) and dimensions to fit within the outer liquid barrier cover (24) and is placed within the outer liquid barrier cover (24), whereby the outer liquid barrier cover (24) and the inner absorption receptacle (25) are provided with the concurring orifices (22) at the same place for receiving the penis.

2. The men's briefs according to claim 1, **characterized in that** the inner absorption receptacle (25) is disposable or washable, wherein an upper end of the outer liquid barrier cover (24) is provided with an opening (26) for inserting the inner absorption receptacle (25).

3. The men's briefs according to claim 1, **characterized in that** the removable incontinence receptacle (G) is disposable, wherein the outer liquid barrier cover (24) and the inner absorption receptacle (25) are on their edges mutually firmly connected.

4. The men's briefs according to any of preceding claims, **characterized in that** the orifice (22) is circular, U-shaped, or of any other shape suitable for receiving the penis.

5. The men's briefs according to any of preceding claims, **characterized in that** the orifice (22) is placed in the removable incontinence receptacle's (G) center line.

6. The men's briefs according to any of preceding claims, **characterized in that** the orifice (19) is placed in the men's briefs center line.

7. The men's briefs according to claim 1, **characterized in that** the elastic means are tightened by the means (21) suitable for adjustment of the adjustable orifice (19).

8. The men's briefs according to any of preceding claims, **characterized in that** the removable incontinence receptacle (G) is provided with a detachable means (23, 23') for detachably connecting the removable incontinence receptacle (G) to the front inner insert (B') and/or the front outer insert (B).

9. A removable incontinence receptacle (G) for use in the men's briefs according to claim 1, the removable incontinence receptacle (G) being in the form of a three-dimensional container ensuring retention of the urine within the removable incontinence receptacle (G), the removable incontinence receptacle (G) being defined by a receptacle front panel (H) and a receptacle back panel (I) that are on the edges mutually firmly connected and with an orifice (22) placed on the receptacle front panel (H), whereby the removable incontinence receptacle (G) has a shape same as said front outer and front inner inserts (B, B') of the men's briefs and dimensions to fit within said front outer and front inner inserts (B, B') of the men's briefs in a manner that the orifice (22) of the receptacle front panel (H) is faced to the adjustable orifice (19) of the men's briefs in order that the removable incontinence receptacle (G) receives the penis, whereby the removable incontinence receptacle (G) consists of an outer liquid barrier cover (24) and an inner absorption receptacle (25), the inner absorption receptacle (25) is defined by two planes mutually firmly connected on their edges forming a pocket like receptacle, the inner absorption receptacle (25) having shape same as the outer liquid barrier cover (24) and dimensions to fit within the outer liquid barrier cover (24) and is placed within the outer liquid barrier cover (24), whereby the outer liquid barrier cover (24) and the inner absorption receptacle (25) are provided with the concurring orifices (22) at the same place for receiving the penis.

10. The removable incontinence receptacle (G) according to claim 9, **characterized in that** the inner absorption receptacle (25) is disposable or washable, wherein an upper end of the outer liquid barrier cover (24) is provided with an opening (26) for inserting the inner absorption receptacle (25).

11. The removable incontinence receptacle (G) according to claim 9, **characterized in that** the removable incontinence receptacle (G) is disposable, wherein the outer liquid barrier cover (24) and the inner absorption receptacle (25) are on their edges mutually firmly connected.

12. The removable incontinence receptacle (G) according to claim 9, **characterized in that** the orifice (22) is circular, U-shaped, or of any other shape suitable for receiving the penis.

13. The removable incontinence receptacle (G) according to claims 9 to 12, **characterized in that** the orifice (22) is placed in the removable incontinence receptacle's (G) center line.

14. The removable incontinence receptacle (G) according to claims 9 to 13, **characterized in that** the removable incontinence receptacle (G) is provided with a detachable means (23, 23') for detachably connecting the removable incontinence receptacle (G) to the front inner insert (B') and/or the front outer insert (B) of the men's briefs.

## Patentansprüche

1. Männerunterhose mit einem abgetrennten Raum zur Aufnahme des Penis, umfassend ein entfernbares absorbierendes Inkontinenzaufnahmebehältnis (G), die als kurze oder lange Männerunterhose hergestellt ist, mit einem elastischen Bund, einer Stoffbahn (A), einem vorderen äußeren Einsatz (B) und einem vorderen inneren Einsatz (B`), wobei der abgetrennte Raum zur Aufnahme des Penis durch den vorderen äußeren Einsatz (B) und den vorderen inneren Einsatz (B') gebildet ist; wobei der vordere äußere Einsatz (B) und der vordere innere Einsatz (B') mit der Stoffbahn (A) und einem optionalen Teil (3) verbunden sind, um die Männerunterhose auszubilden, wobei der vordere innere Einsatz (B') eine Form und Abmessungen gleich wie der vordere äußere Einsatz (B) aufweist, wobei der vordere innere Einsatz (B') durch zwei überlappende vernähte Teile (C) und (D) mit einem gekrümmten Saum (17) bzw. einem gekrümmten Saum (18) gebildet ist, wobei die gekrümmten Säume (17) und (18) ein elastisches Mittel aufweisen, um den Penis verstellbar zu umgeben, wobei die gekrümmten Säume (17) und (18) eine kreisähnliche verstellbare Öffnung (19) zum Einführen des Penis in den abgetrennten Raum ausbilden, wobei die verstellbare Öffnung (19) den Penis umgibt, so dass dessen Herausfallen aus dem abgetrennten Raum verhindert wird, **dadurch gekennzeichnet dass** die Männerunterhose ferner mit dem entfernbaren Inkontinenzaufnahmebehältnis (G) in Form eines dreidimensionalen Behälters versehen ist, der gewährleistet, dass der Urin innerhalb des entfernbaren Inkontinenzaufnahmebehältnisses (G) zurückgehalten wird, wobei das entfernbare Inkontinenzaufnahmebehältnis (G) durch eine Aufnahmebehältnisvorderstoffbahn (H) und eine Aufnahmebehältnisrückstoffbahn (I) definiert ist, die an den Kanten fest miteinander verbunden sind, und mit einer Öffnung (22) versehen ist, die auf der Aufnahmebehältnisvorderstoffbahn (H) platziert ist, wobei das entfernbare Inkontinenzaufnahmebehältnis (G) durch eine durch Säume (11, 5) ausgebildete Laibung innerhalb des vorderen äußeren Einsatzes (B) und des vorderen inneren Einsatzes (B`) platziert ist, wobei das entfernbare Inkontinenzaufnahmebehältnis (G) eine gleiche Form wie der vordere äußere und der vordere innere Einsatz (B, B') aufweist und, wie in der Beschreibung definiert, Abmessungen aufweist, um in den vorderen äußeren und vorderen inneren Einsatz (B, B') zu passen, so dass die Öffnung (22) der Aufnahmebehältnisvorderstoffbahn (H) der verstellbaren Öffnung (19) zugewandt ist, damit das entfernbare Inkontinenzaufnahmebehältnis (G) den Penis aufnimmt, wobei das entfernbare Inkontinenzaufnahmebehältnis (G) aus einer äußeren Flüssigkeitsbarrierenabdeckung (24) und einem inneren Absorptionsaufnahmebehältnis (25) besteht, wobei das innere Absorptionsaufnahmebehältnis (25) durch zwei Ebenen definiert ist, die an ihren Kanten fest miteinander verbunden sind, wobei sie ein taschenähnliches Aufnahmebehältnis ausbilden, wobei das innere Absorptionsaufnahmebehältnis (25) eine gleiche Form wie die äußere Flüssigkeitsbarrierenabdeckung (24) und Abmessungen aufweist, um in die äußere Flüssigkeitsbarrierenabdeckung (24) zu passen, und innerhalb der äußeren Flüssigkeitsbarrierenabdeckung (24) platziert ist, wobei die äußere Flüssigkeitsbarrierenabdeckung (24) und das innere Absorptionsaufnhamebehältnis (25) an der gleichen Stelle mit den übereinstimmenden Öffnungen (22) zur Aufnahme des Penis versehen sind.

2. Männerunterhose nach Anspruch 1, **dadurch gekennzeichnet dass** das innere Absorptionsaufnahmebehältnis (25) für den Einmalgebrauch oder waschbar ist, wobei ein oberes Ende der äußeren Flüssigkeitsbarrierenabdeckung (24) mit einer Öffnung (26) zum Einsetzen des inneren Absorptionsaufnahmebehältnisses (25) versehen ist.

3. Männerunterhose nach Anspruch 1, **dadurch gekennzeichnet dass** das entfernbare Inkontinenzaufnahmebehältnis (G) für den Einmalgebrauch ist, wobei die äußere Flüssigkeitsbarrierenabdeckung (24) und das innere Absorptionsaufnahmebehältnis (25) an ihren Kanten fest miteinander verbunden sind.

4. Männerunterhose nach einem der vorstehenden Ansprüche, **dadurch gekennzeichnet dass** die Öffnung (22) kreisförmig oder U-förmig ist oder eine beliebige andere Form aufweist, die sich zur Aufnahme des Penis eignet.

5. Männerunterhose nach einem der vorstehenden Ansprüche, **dadurch gekennzeichnet dass** die Öffnung (22) in der Mittellinie des entfernbaren Inkontinenzaufnahmebehältnisses (G) platziert ist.

6. Männerunterhose nach einem der vorstehenden Ansprüche, **dadurch gekennzeichnet dass** die Öffnung (19) in der Mittellinie der Männerunterhose platziert ist.

7. Männerunterhose nach Anspruch 1, **dadurch gekennzeichnet dass** das elastische Mittel durch das Mittel (21) verengt wird, das sich zum Verstellen der verstellbaren Öffnung (19) eignet.

8. Männerunterhose nach einem der vorstehenden Ansprüche, **dadurch gekennzeichnet dass** das entfernbare Inkontinenzaufnahmebehältnis (G) mit einem lösbaren Mittel (23, 23') zum lösbaren Verbinden des entfernbaren Inkontinenzaufnahmebehältnisses (G) mit dem vorderen inneren Einsatz (B') und/oder dem vorderen äußeren Einsatz (B) versehen ist.

9. Entfernbares Inkontinenzaufnahmebehältnis (G) zur Verwendung in der Männerunterhose nach Anspruch 1, wobei das entfernbare Inkontinenzaufnahmebehältnis (G) in Form eines dreidimensionalen Behälters vorliegt, der gewährleistet, dass der Urin innerhalb des entfernbaren Inkontinenzaufnahmebehältnisses (G) zurückgehalten wird, wobei das entfernbare Inkontinenzaufnahmebehältnis (G) durch eine Aufnahmebehältnisvorderstoffbahn (H) und eine Aufnahmebehältnisrückstoffbahn (I) definiert ist, die an den Kanten fest miteinander verbunden sind, und mit einer Öffnung (22) versehen ist, die auf der Aufnahmebehältnisvorderstoffbahn (H) platziert ist, wobei das entfernbare Inkontinenzaufnahmebehältnis (G) eine gleiche Form wie der vordere äußere und der vordere innere Einsatz (B, B') der Männerunterhose und Abmessungen aufweist, um in den vorderen äußeren und vorderen inneren Einsatz (B, B') der Männerunterhose zu passen, so dass die Öffnung (22) der Aufnahmebehältnisvorderstoffbahn (H) der verstellbaren Öffnung (19) der Männerunterhose zugewandt ist, damit das entfernbare Inkontinenzaufnahmebehältnis (G) den Penis aufnimmt, wobei das entfernbare Inkontinenzaufnahmebehältnis (G) aus einer äußeren Flüssigkeitsbarrierenabdeckung (24) und einem inneren Absorptionsaufnahmebehältnis (25) besteht, wobei das innere Absorptionsaufnahmebehältnis (25) durch zwei Ebenen definiert ist, die an ihren Kanten fest miteinander verbunden sind, wobei sie ein taschenähnliches Aufnahmebehältnis ausbilden, wobei das innere Absorptionsaufnahmebehältnis (25) eine gleiche Form wie die äußere Flüssigkeitsbarrierenabdeckung (24) und Abmessungen aufweist, um in die äußere Flüssigkeitsbarrierenabdeckung (24) zu passen, und innerhalb der äußeren Flüssigkeitsbarrierenabdeckung (24) platziert ist, wobei die äußere Flüssigkeitsbarrierenabdeckung (24) und das innere Absorptionsaufnhamebehältnis (25) an der gleichen Stelle mit den übereinstimmenden Öffnungen (22) zur Aufnahme des Penis versehen sind.

10. Entfernbares Inkontinenzaufnahmebehältnis (G) nach Anspruch 9, **dadurch gekennzeichnet dass** das innere Absorptionsaufnahmebehältnis (25) für den Einmalgebrauch oder waschbar ist, wobei ein oberes Ende der äußeren Flüssigkeitsbarrierenabdeckung (24) mit einer Öffnung (26) zum Einsetzen des inneren Absorptionsaufnahmebehältnisses (25) versehen ist.

11. Entfernbares Inkontinenzaufnahmebehältnis (G) nach Anspruch 9, **dadurch gekennzeichnet dass** das entfernbare Inkontinenzaufnahmebehältnis (G) für den Einmalgebrauch ist, wobei die äußere Flüssigkeitsbarrierenabdeckung (24) und das innere Absorptionsaufnahmebehältnis (25) an ihren Kanten fest miteinander verbunden sind.

12. Entfernbares Inkontinenzaufnahmebehältnis (G) nach Anspruch 9, **dadurch gekennzeichnet dass** die Öffnung (22) kreisförmig oder U-förmig ist oder eine beliebige andere Form aufweist, die sich zur Aufnahme des Penis eignet.

13. Entfernbares Inkontinenzaufnahmebehältnis (G) nach den Ansprüchen 9 bis 12, **dadurch gekennzeichnet dass** die Öffnung (22) in der Mittellinie des entfernbaren Inkontinenzaufnahmebehältnisses (G) platziert ist.

14. Entfernbares Inkontinenzaufnahmebehältnis (G) nach einem der Ansprüche 9 bis 13, **dadurch gekennzeichnet dass** das entfernbare Inkontinenzaufnahmebehältnis (G) mit einem lösbaren Mittel (23, 23') zum lösbaren Verbinden des entfernbaren Inkontinenzaufnahmebehältnisses (G) mit dem vorderen inneren Einsatz (B') und/oder dem vorderen äußeren Einsatz (B) der Männerunterhose versehen ist.

## Revendications

1. Slip pour homme avec un espace séparé pour recevoir le pénis comprenant un réceptacle amovible absorbant l'incontinence (G), réalisé sous la forme d'un slip à jambes courtes ou longues, avec une ceinture flexible, un panneau (A), un insert extérieur avant (B) et un insert intérieur (B'), dans lequel l'espace séparé pour recevoir le pénis est formé par l'insert extérieur avant (B) et l'insert intérieur avant (B') ; l'insert extérieur avant (B) et l'insert intérieur avant (B') étant reliés au panneau (A) et à un élément facultatif (3) de manière à former le slip pour homme, dans lequel l'insert intérieur avant (B') a une forme et des dimensions identiques à celles de l'insert extérieur avant (B), où l'insert intérieur avant (B') est formé de deux parties cousues (C) et (D) se chevauchant ayant un ourlet arrondi (17) et un ourlet arrondi (18), respectivement, les ourlets arrondis (17) et (18) sont équipés d'un moyen élastique pour encercler de manière réglable le pénis, où lesdits ourlets arrondis (17) et (18) forment un orifice ajustable de type circulaire (19) pour insérer le pénis dans l'espace séparé, l'orifice réglable (19) encercle le pénis d'une manière qui l'empêche de tomber hors de l'espace séparé, **caractérisé en ce que** lesdits slips pour homme sont en outre munis du réceptacle amovible pour incontinence (G) se présentant sous la forme d'un contenant assurant la rétention de l'urine à l'intérieur du réceptacle amovible pour incontinence (G), le réceptacle amovible pour incontinence (G) est défini par un panneau avant de réceptacle (H) et un panneau arrière de réceptacle (I) qui sont mutuellement situés sur les bords fermement connectés et avec un orifice (22) placé sur le panneau avant du réceptacle (H), le réceptacle amovible pour incontinence (G) est placé à l'intérieur de l'insert extérieur avant (B) et de l'insert intérieur avant (B') à travers une embrasure formée par des ourlets (11, 5), de sorte que le réceptacle amovible pour incontinence (G) a la même forme que lesdits inserts extérieur et intérieur avant (B, B') et tel que défini dans la description, des dimensions permettant de s'adapter dans lesdits inserts extérieur et intérieur avant (B, B') de manière que l'orifice (22) du panneau avant du réceptacle (H) soit tourné vers l'orifice réglable (19) afin que le réceptacle amovible pour incontinence (G) reçoive le pénis, dans lequel le réceptacle amovible pour incontinence (G) se compose d'un cache extérieur formant barrière aux liquides (24) et d'un réceptacle d'absorption interne (25), le réceptacle d'absorption interne (25) est défini par deux plans fermement connectés mutuellement sur leurs bords formant un réceptacle en forme de poche, le réceptacle d'absorption interne (25) ayant la même forme que le cache externe formant barrière aux liquides (24) et des dimensions permettant de s'adapter à l'intérieur du cache externe formant barrière aux liquides (24) et est placé à l'intérieur du cache externe formant barrière aux liquides (24), de sorte que le cache externe formant barrière aux liquides (24) et le réceptacle d'absorption interne (25) sont pourvus des orifices concordants (22) au même endroit pour recevoir le pénis.

2. Slip pour homme selon la revendication 1, **caractérisé en ce que** le réceptacle d'absorption intérieur (25) est jetable ou lavable, dans lequel une extrémité supérieure du cache extérieur formant barrière aux liquides (24) est pourvue d'une ouverture (26) pour insérer le réceptacle d'absorption intérieur (24).

3. Slip pour homme selon la revendication 1, **caractérisée en ce que** le réceptacle amovible pour incontinence (G) est jetable, dans lequel le cache extérieur formant barrière aux liquides (24) et le réceptacle d'absorption intérieur (25) sont mutuellement fermement connectés sur leurs bords.

4. Slip pour homme selon l'une quelconque des revendications précédentes, **caractérisé en ce que** l'orifice (22) est circulaire, en forme de U ou de toute autre forme adaptée pour recevoir le pénis.

5. Slip pour homme selon l'une quelconque des revendications précédentes, **caractérisé en ce que** l'orifice (22) est placé dans la ligne médiane du réceptacle amovible pour incontinence (G).

6. Slip pour homme selon l'une quelconque des revendications précédentes, **caractérisé en ce que** l'orifice (19) est placé dans la ligne médiane du slip pour homme.

7. Slip pour homme selon la revendication 1, **caractérisé en ce que** les moyens élastiques sont serrés par des moyens (21) adaptés au réglage de l'orifice réglable (19).

8. Slip pour homme selon l'une quelconque des revendications précédentes, **caractérisé en ce que** le réceptacle amovible pour incontinence (G) est pourvu d'un moyen amovible (23, 23') pour relier de manière amovible le réceptacle amovible pour incontinence (G) à l'insert intérieur avant (B') et/ou l'insert extérieur avant (B).

9. Réceptacle amovible pour incontinence (G) destiné à être utilisé dans des slips pour homme selon la revendication 1, le réceptacle amovible pour incontinence (G) se présentant sous la forme d'un récipient tridimensionnel assurant la rétention de l'urine à l'intérieur du réceptacle amovible pour incontinence (G), le réceptacle amovible pour incontinence (G) étant défini par un panneau avant de réceptacle (H) et un panneau arrière de réceptacle (I) qui sont mutuellement fermement connectés sur les bords et avec un orifice (22) placé sur le panneau avant de réceptacle (H), de sorte que le réceptacle amovible pour incontinence (G) a la même forme que lesdits inserts extérieur et intérieur avant (B, B') du slip pour homme et des dimensions permettant de s'adapter dans lesdits inserts extérieur et intérieur avant (B, B') du slip pour homme de manière à ce que l'orifice (22) du panneau avant du réceptacle (H) soit face à l'orifice réglable (19) du slip pour homme afin que le réceptacle amovible pour incontinence (G) reçoive le pénis, le réceptacle amovible pour incontinence (G) se compose d'un cache extérieur formant barrière aux liquides (24) et d'un réceptacle d'absorption intérieur (25), le réceptacle d'absorption intérieur (25) est défini par deux plans mutuellement fermement connectés sur leurs bords formant un réceptacle en forme de poche, le réceptacle d'absorption intérieur (25) ayant la même forme que le cache extérieur formant barrière aux liquides (24) et des dimensions permettant de s'adapter à l'intérieur du cache extérieur formant barrière aux liquides (24) et est placé à l'intérieur du cache extérieur formant barrière aux liquides (24), de sorte que le cache extérieur formant barrière aux liquides (24) et le réceptacle d'absorption intérieur (25) sont pourvus des orifices concordants (22) au même endroit pour recevoir le pénis.

10. Réceptacle amovible pour incontinence (G) selon la revendication 9, **caractérisé en ce que** le réceptacle d'absorption intérieur (25) est jetable ou lavable, dans lequel une extrémité supérieure du cache extérieur formant barrière aux liquides (24) est pourvue d'une ouverture (26) pour insérer le réceptacle d'absorption intérieur (25).

11. Réceptacle amovible pour incontinence (G) selon la revendication 9, **caractérisé en ce que** le réceptacle amovible pour incontinence (G) est jetable, dans lequel le cache extérieur formant barrière aux liquides (24) et le réceptacle d'absorption intérieur (25) sont mutuellement fermement connectés sur leurs bords.

12. Réceptacle amovible pour incontinence (G) selon la revendication 9, **caractérisé en ce que** l'orifice (22) est circulaire, en forme de U, ou de toute autre forme adaptée pour recevoir le pénis.

13. Réceptacle amovible pour incontinence (G) selon les revendications 9 à 12, **caractérisé en ce que** l'orifice (22) est placé dans la ligne médiane du réceptacle amovible pour incontinence (G).

14. Réceptacle amovible pour incontinence (G) selon les revendications 9 à 13, **caractérisé en ce que** le réceptacle amovible pour incontinence (G) est pourvu d'un moyen pouvant être détaché (23, 23') pour connecter de façon amovible le réceptacle amovible pour incontinence (G) à l'insert intérieur avant (B') et/ou l'insert extérieur avant (B) du slip pour homme.
